Europäisches Patentamt

(19) European Patent Office (11) Publication number: **0 071 238**

Office européen des brevets **B1**

(12) **EUROPEAN PATENT SPECIFICATION**

(45) Date of publication of patent specification: **10.09.86** (51) Int. Cl.⁴: **C 07 C 43/04, C 07 C 41/06**

(21) Application number: **82106760.0**

(22) Date of filing: **26.07.82**

(54) **Process for the preparation of methyl tert-butyl ether.**

(30) Priority: **27.07.81 US 287458**

(43) Date of publication of application:
**09.02.83 Bulletin 83/06**

(45) Publication of the grant of the patent:
**10.09.86 Bulletin 86/37**

(84) Designated Contracting States:
**BE DE FR GB IT**

(56) References cited:
**FR-A-2 450 799**
**GB-A-2 034 311**
**US-A-4 182 913**

(73) Proprietor: **PHILLIPS PETROLEUM COMPANY**
**5th and Keeler**
**Bartlesville Oklahoma 74004 (US)**

(72) Inventor: **Makovec, Donald John**
**5857 S.E.Meadowcrest**
**Bartlesville Oklahoma 74003 (US)**

(74) Representative: **Dost, Wolfgang, Dr.rer.nat.,**
**Dipl.-Chem. et al**
**Patent- und Rechtsanwälte Bardehle-**
**Pagenberg-Dost-Altenburg & Partner Postfach**
**86 06 20**
**D-8000 München 86 (DE)**

## Description

Field of the invention

The present invention relates to the production of methyl-tert-butyl ether from isobutylene.

Background of invention

In view of the growing need to limit the use of lead-containing additives as octane improvers for gasoline, there is an increasing need for more environmentally safe octane-improving additives. One of the more promising additives is methyl tert-butyl ether (MTBE).

In the British Patent 2 048 247 a process for continuous preparation of MTBE is described wherein isobutylene and methanol are reacted in contact with an anion exchange resin. A portion of the reaction product is recycled. A broad range of isobutylene to methanol ratios is disclosed in this patent and the reaction temperature is disclosed to be in the range of 60 to 100°C.

· In the British Patent 2 034 311 a process for the production of MTBE by reacting a steam-cracking C$_4$ cut with methanol is described. This process uses two reaction zones and a recycle of effluent from the first reaction zone to the inlet thereof. The reaction temperatures for the two reaction zones are described to be in the range of 80 to 100°C for the first reaction zone.

US Patent 4 182 913 describes a method for producing MTBE in one reaction zone.

There is a continued need for processes for producing MTBE in high yield and at low cost.

It has long been known that MTBE can be produced from the reaction of isobutylene and methanol. See Leum et al, U. S. patent 2,480,940. In a commercial scale MTBE plant, it is obviously desirable to maximize the production of substantially pure MTBE without the need for numerous or expensive separation steps. It has previously been suggested that the MTBE yield could be increased by using excess amounts of either the isobutene or the methanol. Such techniques have, however, led to separation problems or undesirable by-products which adversely affect catalyst life.

An object of the present invention is to provide a method for producing MTBE in a form that is readily separated into a substantially pure MTBE product.

Still another object of the present invention is to provide a process in which the catalyst activity can be maintained over long periods of time.

Other aspects, objects and advantages of the present invention will be apparent from the following disclosure and the attached Figures.

Summary of the invention

In accordance with the present invention, methyl tertiary butyl ether is prepared by reacting isobutylene and methanol wherein said reaction is carried out in two stages in a first and a second reaction zone each comprising a fixed bed of acid ion exchange resin and kept under substantially adiabatic conditions and a pressure sufficient to maintain the reactants in the liquid phase, wherein a fresh feedstream comprising isobutylene and methanol is fed through said first reaction zone at an LHSV of 2 to 5, yielding an isobutylene conversion of at least 90 weight percent and at least as great as and preferably greater than it would be under the same inlet temperature, adiabatic and fresh feed LHSV conditions without effluent recycling and effluent is recycled from and through said first reaction zone along with said fresh feedstream, the recycle ratio of effluent from said first reaction zone being not greater than 14, and unrecycled effluent from said first reaction zone is passed through said second reaction zone with an inlet temperature in the range of 38 to 43°C and at a LHSV in the range of 3 to 7, yielding a conversion of at least 50 weight percent of the remaining isobutylene. The inventive process is characterized in that the molar ratio of methanol to isobutylene in the combined feeds entering said first reaction zone is in the range of 0.99:1 to 1.02:1 and the inlet temperature of the feeds is in the range of 49 to 60°C.

The process of this invention in a preferred embodiment comprises carrying out said reaction in two stages by feeding a fresh feedstream comprising isobutylene and methanol through a first reaction zone comprising a fixed bed of acid ion exchange resin under substantially adiabatic conditions and a pressure sufficient to maintain the reactants in the liquid phase and recycling effluent from said first reaction zone through said first reaction zone along with said fresh feedstream wherein the fresh feedstream contains no more than about 25 weight percent, preferably 15—20 weight percent, isobutylene on a methanol free basis, the molar ratio of methanol to isobutylene entering said first reactor is about 1/1, said fresh feedstream and said recycle stream are passed into said first reaction zone at a temperature of about 120°F (49°C) to about 140°F (60°C) the LHSV of said fresh feedstream is in the range of about 3 to about 5, the recycle ratio is about 2 to about 6; and passing unrecycled effluent from said first reaction zone through a second reaction zone comprising a fixed bed of acid ion exchange resin under substantially adiabatic conditions and a pressure sufficient to maintain the reactants in the liquid phase wherein said unrecycled effluent is passed into said catalyst bed of said second reaction zone at a temperature of about 100°F (38°C) to about 110°F (43°C) and a LHSV of about 3 to 7.

In a preferred embodiment of the present invention, effluent from said first reaction zone still containing substantially all its MTBE is passed through a second reaction zone comprising a fixed bed of acid ion exchange resin under substantially adiabatic conditions and a pressure sufficient to maintain a liquid phase reaction under conditions which maximize the overall conversion of isobutene obtained from the two reaction steps.

The term "substantially adiabatic conditions" is used herein to indicate that no cooling of the reactor is employed other than the small amount that would normally occur under the ambient conditions.

Brief description of the figures

Figure 1 is a graphical illustration of the effect of recycle ratio and fresh feedrate on isobutene conversion in the first reaction zone.

Figure 2 is a graphical illustration of the effect of inlet temperature and recycle ratio on isobutene conversion in the first reaction zone.

Figure 3 is a graphical illustration of the effect of inlet temperature and space velocity on isobutene conversion in the second reaction zone.

Figures 4 and 5 are graphical illustrations of the effect of temperature, recycle ratio, and fresh feed LHSV on isobutene conversion in the first reaction zone for a fresh feedstream containing 43 weight percent isobutene.

Figure 6 is a graphical illustration of the effect of temperature, recycle ratio, and fresh feed LHSV on isobutene conversion in the first reaction zone for a fresh feed containing 60 weight percent isobutene.

Figure 7 is a graphical illustration comprising the results obtained in the first reaction zone for fresh feeds having various levels of isobutene.

Figures 8 and 9 are graphical illustrations comprising the results obtained in the first reaction zone for different methanol to isobutene ratios.

Figure 10 is a schematic illustration of a process employing the present invention to obtain high yields of relatively pure MTBE products with minimal separation expenditure.

Detailed description of the invention

The catalyst employed in the present invention is a macroreticular acid ion exchange resin of the type known to be suitable for catalyzing the reaction of methanol and isobutene to MTBE. A typical example is Amberlyst™15 available from the Rohm and Haas Company.

Any suitable isobutene-containing feed can be employed in the present invention. Typically, the isobutene feed is a $C_4$ fraction from a steam or catalytic cracking operation. Preferably, the feed is subjected to a butadiene extraction step to remove any substantial amounts of butadiene prior to reaction with the methanol. Typically, the fresh feedstream on a methanol free basis contains at least 10 weight percent isobutene and no more than 60 weight percent isobutene. Most preferably, the fresh feedstream contains no more than 25 weight percent isobutene on a methanol-free basis. The use of fresh feedstream having more than 25 weight percent isobutene often results in final product streams containing more than 1.5 weight percent methanol. If the amount of methanol in the final product stream is kept below 1.5 weight percent, it is possible to separate substantially all of the methanol from the MTBE by distilling the methanol overhead with the unreacted $C_4$'s. If product streams from the second reactor are obtained that have much more than 1.5 weight percent methanol, the methanol does not completely azeotrope overhead with the unreacted $C_4$'s and will contaminate the MTBE product which will necessitate the use of special additional separation techniques to obtain an MTBE product substantially free of methanol.

As will be seen from the examples that follow, the LHSV of the fresh feed and the recycle rate that is employed in the first reaction zone can vary over a wide range depending upon the inlet temperature, the weight percent isobutene in the feed, the temperature conditions, and the level of isobutene conversion desired. The term LHSV as used herein refers to the volumes of the particular stream that is passed through the reactor per volume of catalyst per hour wherein the volume of the catalyst refers to the dry volume of the catalyst. The recycle ratio refers to the volumes of recycle stream per volume of fresh feed. The LHSV for the fresh feed to the first reaction zone is 2 to 5. The recycle ratio is generally no greater than 14, more normally no greater than 5. Obviously, the best conditions are those which result in isobutene conversion that is greater than the conversion that would be obtained at the same conditions of temperature and fresh feed LHSV but without the recycle. It is typically desired that the recycle be employed at such a rate that the maximum hot spot in the catalyst bed is at a temperature no greater than 25°F (14°C) above the inlet temperature, with lower hot spot temperatures being more desirable for the higher inlet temperatures.

The molar ratio of methanol to isobutene in the stream resulting from the combination of the fresh feed and the recycle should be about 1/1. Methanol to isobutene ratios in the range of 0.99 to 1.02 have been found quite suitable. Since occasionally some of the isobutene in the fresh feed may dimerize or react with small amounts of water present to form tert-butyl alcohol, it is generally preferred that the molar ratio of methanol to isobutene in the fresh feed be not greater than 1/1, so that the methanol to isobutene ratio in the combined streams will be maintained to about 1/1.

The unrecycled effluent from the first reaction zone is passed through the second reaction zone at any LHSV that will provide at least 50 weight percent conversion of the remaining isobutene. Generally, it is desirable to employ a LHSV in the second reaction zone which results in a catalyst hot spot no more than 10°F (6°C) hotter than the inlet temperature, more preferably no more than about 5°F (3°C) hotter than the inlet temperature. The LHSV of the unrecycled effluent passed into the second reaction zone will be in the range of 3 to 7.

A further understanding of the present invention and its advantages will be provided by the following examples which summarize the results obtained using pilot plant scale etherization reactions.

The evaluations were made using a two inch I.D. carbon steel reactor insulated to provide substantially adiabatic conditions. The reactor was filled with 780 cc of dry Amberlyst™15 ion exchange resin available from Rohm and Haas. The Amberlyst™15 was a strongly acidic macroreticular cation exchange resin comprising particles over 90 percent of which were 20—50 mesh. The bulk density of the dry catalyst was 38 pounds/cubit foot (0.61 g/cc). In as much as the dry catalyst swells when wetted, it is noted that the LHSV used herein (i.e., the volumes of liquid feed per volume of catalyst per hour), are based on the dry weight of the catalyst.

Example I

A typical hydrocarbon feed was mixed with enough methanol to give a fresh feed having a methanol/isobutene feed ratio of 1/1. The composition of the fresh feed was:

|              | Weight, % |
| ------------ | --------- |
| Propane      | 0.28      |
| Propene      | 0.20      |
| Isobutane    | 23.25     |
| n-Butane     | 19.85     |
| Butene-1     | 11.87     |
| Isobutene    | 13.10     |
| t-Butene-2   | 13.64     |
| c-Butene-2   | 9.86      |
| 1,3-Butadiene| 0.22      |
| Isopentane   | 0.25      |
| Methanol     | 7.48      |

The feedstream was then used to evaluate the effect of recycle on the conversion of isobutene. An automatic level control scheme was used to purge inert gases and insure a liquid-full reactor. Reactor effluent was cooled, metered, and then reheated along with fresh feed in an electric furnace, controlling heat input to the furnace to obtain the desired inlet temperature. The pressure was maintained in the range of about 150 to about 170 psig (10.3—11.8 bar) to insure liquid phase reaction.

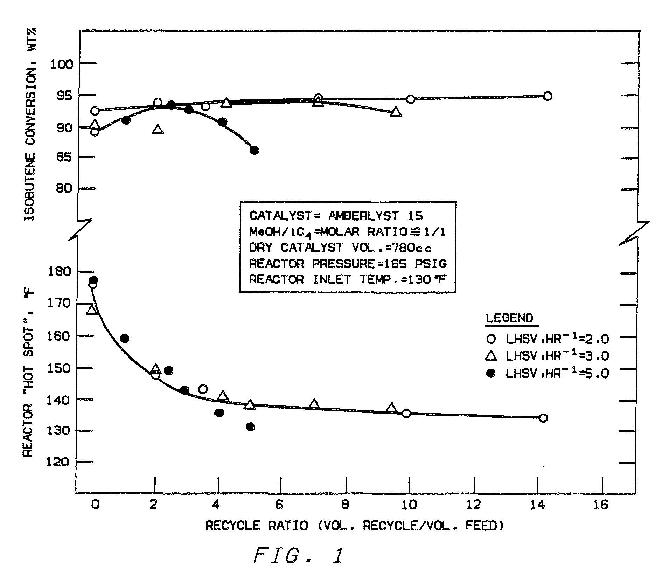

The effect of varying the LHSV of fresh feed and the recycle ratio, i.e., volumes of reactor effluent recycle per volumes of fresh feed, is shown in Figure 1. Under the noted conditions, the temperature buildup in the reactor decreased with increased recycle ratio. More importantly the data shows that the use of recycle can result in higher isobutene conversion than can be obtained without recycle. The term isobutene conversion as used herein refers to the isobutene conversion based on the isobutene.

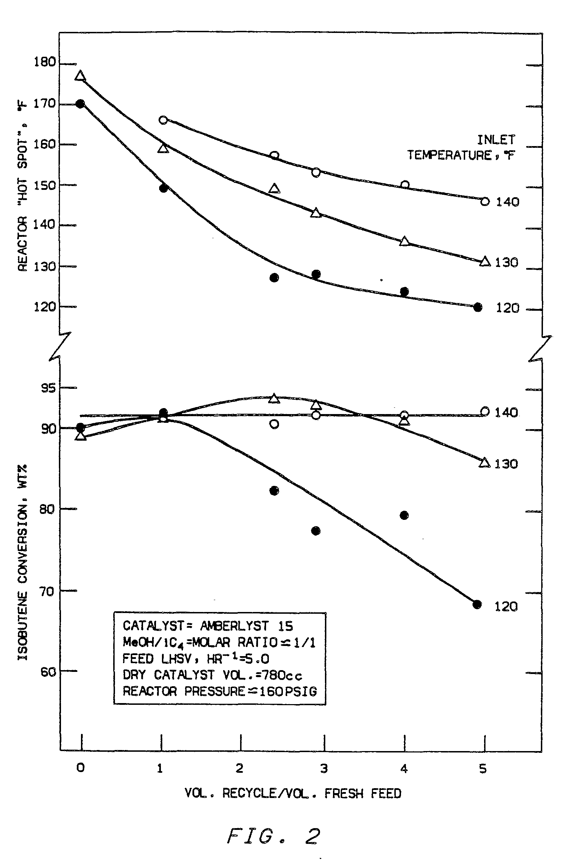

The effect of varying the inlet temperature is shown in Figure 2. Again the reactor "hot spot" was lower at higher recycle ratios. It further shows that at an inlet temperature of 120°F (49°C) some improvement in isobutene conversion is obtained at recycle ratios lower than 1. At inlet temperatures of 130 and 140°F (54 and 60°C) high conversions of isobutene were obtained over a broad range of recycle ratios. Optimum conversion was obtained using an inlet temperature of 130°F (54°C) and recycle ratios in the range of about 1.0 to about 3.5.

Example II

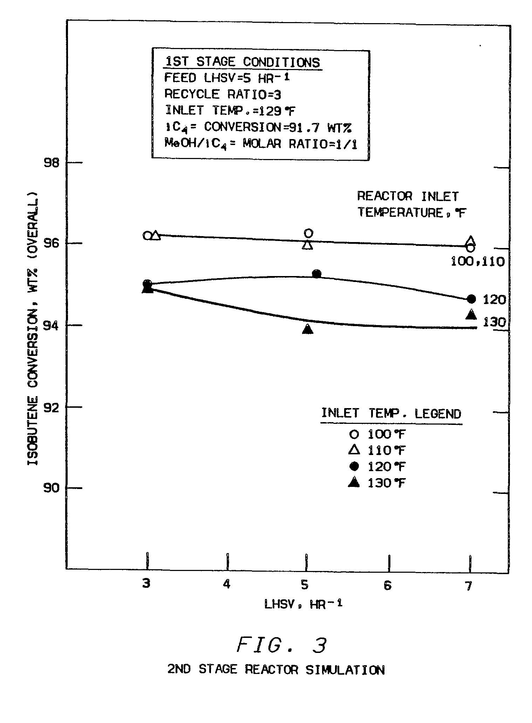

Effluent from a reaction carried out in a first reactor as set forth in Example I was passed through a second reaction zone comprising a bed of Amberlyst™15 to determine the effect of the inlet temperature on tha total isobutene conversion. The fresh feed supplied to the first reactor was as indicated in Example I. No recycle was employed in the second reactor and the second reactor was operated under substantially adiabatic conditions. The operating conditions and results are summarized in Figure 3.

The results show that when the LHSV is in the range of 3 to 7, the best overall isobutene conversion is obtained when the inlet temperature to the second reactor is in the range of about 100°F to about 110°F (37 to 43°C).

Example III

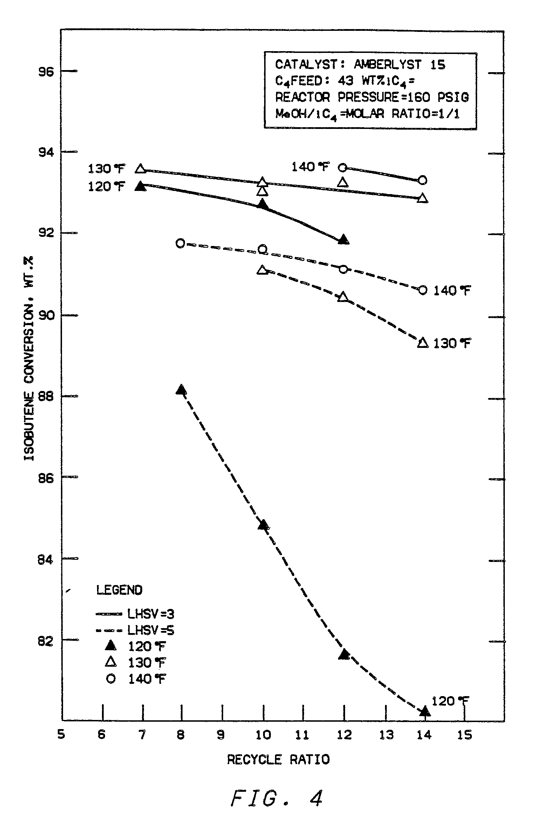

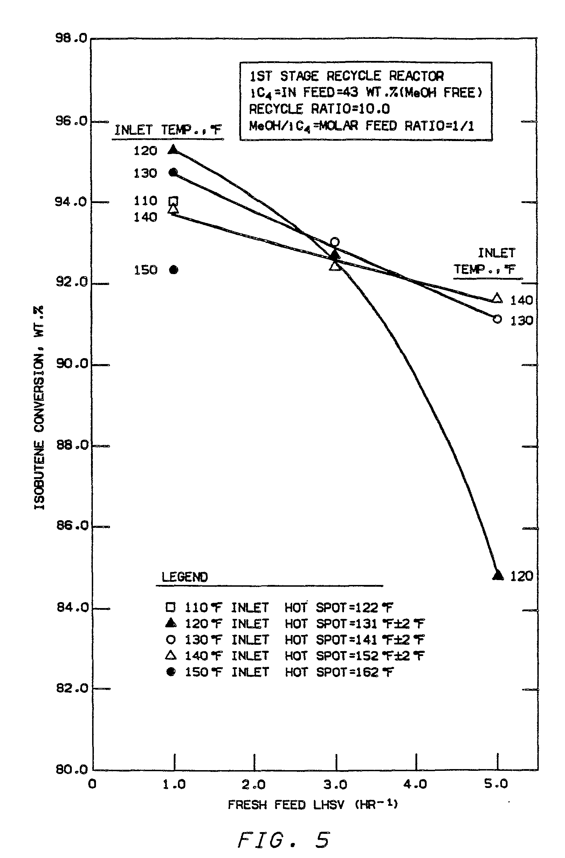

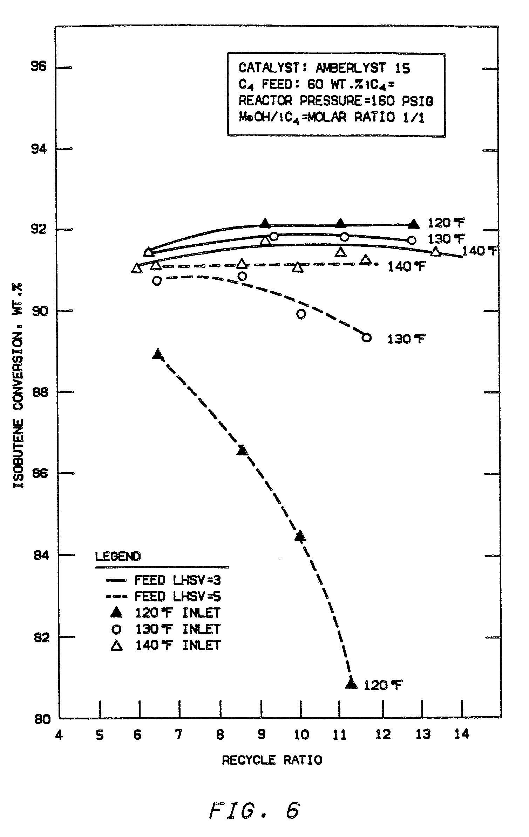

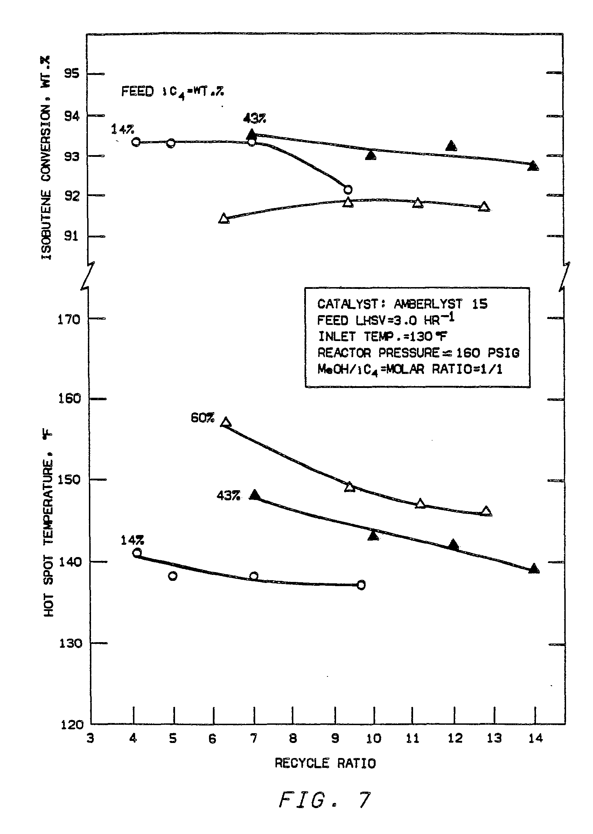

Using the recycle pilot plant reactor as in Example I, a series of runs were made using fresh feeds containing either 43 weight percent or 60 weight percent isobutylene. The conditions employed and the results obtained are summarized in Figures 4 to 6. A comparison of these results to those obtained with a 14 weight percent isobutylene feed is provided in Figure 7. The results in Figure 7 that under the given operating conditions increasing the isobutene feed concentration from 14.0 to 60 weight percent (methanol free basis) reduces the isobutene conversion by no more than about 2 weight percent.

Example IV

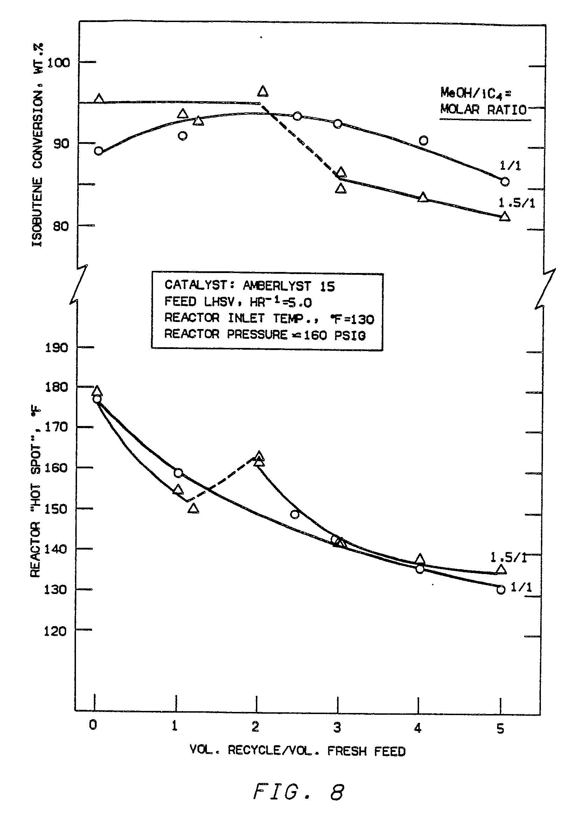

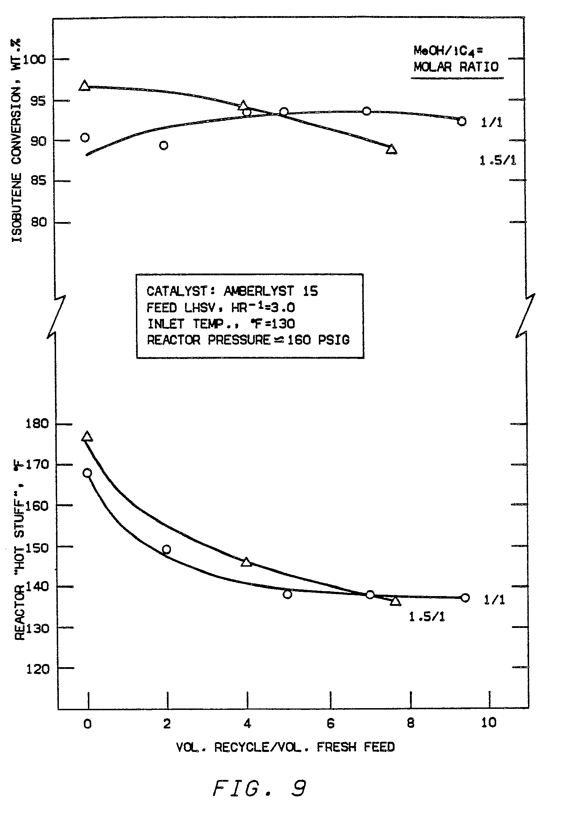

Again using the recycle pilot plant reactor as in Example I, a series of runs were made using a fresh feed having a methanol/isobutylene ratio of 1.5/1. As indicated earlier such a technique has often been suggested as a means of increasing the isobutene conversion. The results are summarized in Figures 8 and 9. The data indicates that while it is true that when no effluent recycle is employed, the excess methanol results in greater isobutylene conversion. However, when recycle is used, greater isobutylene conversion can be obtained using a 1/1 molar ratio. An even more important feature is illustrated by Figure 9 where it is demonstrated that although isobutylene conversion is greater with excess methanol, the weight percent MTBE obtained is much less than when a 1/1 molar ratio is employed. In addition, the use of excess methanol results in an effluent having much higher levels of methanol. The higher levels of methanol are quite significant since high levels of methanol in the effluent can make it impossible to obtain a relatively clean separation of methanol and MTBE by distillation. Figures 8 and 9 thus demonstrate that there is a distinct advantage to carrying out the etherization reaction in the first reaction zone in the manner set forth in this disclosure.

Example V

Again using the recycle pilot plant reactor as in Example I, a series of runs were made using fresh feeds having methanol to isobutene ratios of 0.4/1 or 0.7/1. The results are summarized in Tables I and II.

TABLE I

Effect of space velocity and inlet temperature at a Methanol/Isobutene molar feed ratio of 0.7/1

| Run No. | 111 | 112 | 105 | 106 | 103 | 104 | 109 | 113 | 110 | 107 | 108 |
|---|---|---|---|---|---|---|---|---|---|---|---|
| Conditions | | | | | | | | | | | |
| Fresh Feed LHSV, hr$^{-1}$ | 3 | 5 | 3 | 5 | 3 | 5 | 3 | 3 | 5 | 3 | 5 |
| Recycle Ratio | 0 | 0 | 0 | 0 | 0 | 0 | 2.1 | 4.0 | 2.0 | 2.2 | 2.0 |
| Total LHSV, hr$^{-1}$ | 3 | 5 | 3 | 5 | 3 | 5 | 9.4 | 15.0 | 15.0 | 9.4 | 15.0 |
| Reactor Inlet Temp., °F (°C) | 110 (43) | 110 (43) | 120 (49) | 120 (49) | 130 (54) | 130 (54) | 120 (49) | 120 (49) | 120 (49) | 130 (54) | 130 (54) |
| Reactor Pressure, psig (bar) | 159 (11) | 160 (11) | 160 (11) | 160 (11) | 160 (11) | 160 (11) | 157 (10.8) | 157 (10.8) | 157 (10.8) | 157 (10.8) | 157 (10.8) |
| Results | | | | | | | | | | | |
| iC$_4$=Conversion, Wt % | 78.3 | 75.4 | 77.8 | 75.0 | 77.8 | 75.5 | 76.7 | 79.2 | 74.7 | 76.9 | 75.5 |
| MeOH Conversion, Wt % | 88.3 | 86.8 | 86.4 | 83.3 | 85.0 | 84.8 | 88.3 | 89.8 | 89.1 | 86.2 | 88.9 |
| MTBE in Reactor Eff., Wt. % | 12.8 | 13.0 | 12.6 | 13.1 | 12.1 | 12.3 | 12.9 | 14.2 | 12.9 | 13.1 | 13.1 |
| DIB & MSBE[1] in Reactor Eff., Wt % | — | — | — | — | — | — | — | — | — | — | — |

[1] Diisobutene and methyl secondary butyl ether.

TABLE II
Effect of space velocity and inlet temperature at a
Methanol/Isobutene molar feed ratio of 0.4/1[1]

| Run No. | 117 | 116 | 120 | 121 | 115 | 114 | 118 | 119 |
|---|---|---|---|---|---|---|---|---|
| Conditions | | | | | | | | |
| Fresh Feed LHSV, hr$^{-1}$ | 3 | 5 | 3 | 5 | 3 | 5 | 3 | 5 |
| Recycle Ratio | 0 | 0 | 2.1 | 2.0 | 0 | · 0 | 2.1 | 2.0 |
| Total LHSV, hr$^{-1}$ | 3 | 5 | 9.4 | 15.0 | 3 | 5 | 9.4 | 15.0 |
| Reactor Inlet Temp., °F (°C) | 121 (49) | 121 (49) | 121 (49) | 120 (49) | 131 (55) | 130 (54) | 130 (54) | 130 (54) |
| Reactor Pressure, psig (bar) ·· | 162 (11.2) | 162 (11.2) | 158 (10.9) | 158 (10.9) | 162 (11.2) | 160 (11.0) | 157 (10.8) | 158 (10.9) |
| Results | | | | | | | | |
| iC$_4$=Conversion, Wt % | 85.1 | 84.0 | 61.3 | 51.8 | 86.9 | 82.8 | 73.5 | 63.7 |
| MeOH Conversion, Wt % | 80.3 | 82.9 | 87.0 | 87.3 | 79.4 | 77.5 | 86.3 | 85.7 |
| MTBE in Reactor Eff., Wt % | 5.6 | 4.8 | 6.4 | 6.6 | 2.8 | 4.6 | 6.4 | 6.3 |
| DIB & MSBE[2] in Reactor Eff., Wt. % | 6.3 | 7.3 | 2.7 | 1.6 | 8.8 | 4.2 | 4.1 | 3.5 |

[1] Using single-stage reactor.
[2] Diisobutene and methyl secondary butyl ether.

This data indicates that the lower mole ratios of methanol to isobutylene did not result in isobutylene conversions of 90 weight percent. Further, surprisingly, the lower levels of methanol did not result in a greater weight percent of methanol conversion than the runs using a ratio of about 1/1. Still further, it was noted that as the molar ratio of methanol of isobutylene is decreased, there is an increasing tendency for the formation of undesired diisobutene and methyl secondary butyl ether by-products. The lower molar ratios were also found to affect the activity of the catalyst probably as a result of catalyst fouling by polymer or other undesirable by-products formation.

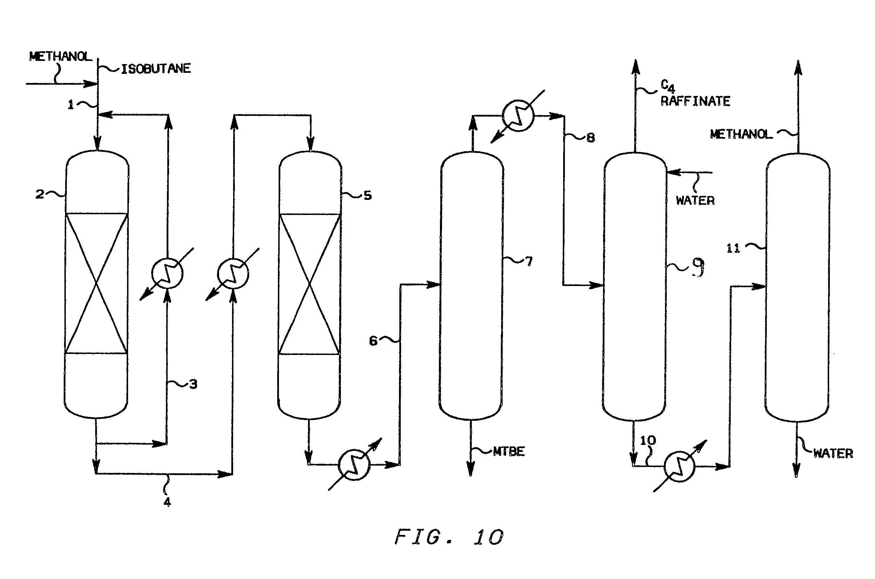

The schematic process flow diagram presented in Figure 10 illustrates a plant designed to produce MTBE using the beneficial results provided by the present invention.

A $C_4$ olefinic stream containing isobutene is combined with methanol and passed through line 1 to a first reactor 2 containing a fixed bed of an acid ion exchange resin such as Amberlyst™15. A portion of the effluent from the reactor 2 is cooled and recycled via line 3 to the inlet of reactor 2. The cooling of the recycled effluent is used to control the temperature of the combined streams at the inlet of reactor 2.

The unrecycled effluent is cooled and passed via line 4 to a reactor 5 also containing a fixed bed of acid ion exchange resin. The effluent from reactor 5 is heated and passed via line 6 to a fractionation column 7 wherein MTBE is separated from methanol and unreacted portions of the $C_4$ feed.

The overhead from fractionator 7 is cooled and passed via line 8 to a water wash column 9 wherein methanol is washed out of the unreacted portions of the $C_4$ feed. The bottoms from the wash column 9 comprising methanol and water is then heated and passed via line 10 to a fractionation column 11 wherein water and methanol are separated. Generally, the fractionation in column 11 can be carried out so as to result in methanol suitable for reuse as the methanol reactant stream that is passed into reactor 2.

In a typical operation, a $C_4$ hydrocarbon cut from a catalytic cracking operation containing about 15 to about 20 weight percent isobutene is combined with methanol to result in a fresh feedstream having a methanol to isobutene ratio of about 1/1. This feedstream is passed via line 1 along with recycle effluent from line 3 through reactor 2. The inlet temperature of the combined streams would typically be about 130°F (54°C). The pressure in reactor 2 under these conditions would typically be about 180 to 235 psia (1.24 to 1.62 MPa absolute). The effluent is recycled via line 3 at such a rate that the temperature of the effluent at the outlet of reactor 2 is in the range of 155 to 160°F (68 to 71°C). Such a fresh feed would be passed through the first reactor at a LHSV of about 3 to 5. The recycle ratio would be in the range of about 2 to about 6.

Unrecycled effluent would be passed via line 4 into the second reactor 5. The unrecycled effluent would be cooled to a temperature in the range of 100 to 110°F (38 to 43°C) and the LHSV of the unrecycled effluent supplied to reactor 5 would be such as to assure that the temperature of effluent of reactor 5 at the outlet has a temperature in the range of about 5 to 10°F (3 to 6°C) greater than that of the inlet temperature of the feed to said reactor 5. The pressure would typically be 170 to 210 psia (1.17 to 1.45 MPa absolute).

The effluent from the second reactor is then heated and passed via line 6 to a distillation column 7. The distillation column is operated under conditions such that methanol and unreacted hydrocarbons are distilled overhead and MTBE is recovered from the bottom in a form substantially free of methanol. Typically, the pressure in the column 7 would be in the range of 85 to 125 psia (0.59 to 0.86 MPa absolute). Typically, the recovered MTBE product will contain essentially no methanol and will be at least about 98 weight percent MTBE, the remainder generally comprising higher boiling hydrocarbons such as pentanes and 1-pentene present in the fresh feed stream.

The overhead from the fractionator 7 is then passed to a methanol extractor 9 wherein countercurrent contact with water separates the methanol and the hydrocarbons. Typical operating conditions for extractor 9 are a temperature of about 100°F (38°C) and a pressure of about 210 psia (1.45 MPa absolute). The $C_4$ raffinate can be recovered and processed as desired for further use.

The methanol-water mixture is passed to fractionator 11 wherein the methanol and water are fractionated. Typically, this fractionator is operated at pressures in the range of about 30 to about 50 psia (0.21 to 0.35 MPa absolute). Generally under such conditions, the methanol that is recovered is sufficiently free of water that it can be used as make-up for the methanol supplied to etherization reactor 2.

## Claims

1. A process for preparing methyl tertiary butyl ether by reacting isobutylene and methanol wherein said reaction is carried out in two stages in a first and a second reaction zone each comprising a fixed bed of acid ion exchange resin and kept under substantially adiabatic conditions and a pressure sufficient to maintain the reactants in the liquid phase, wherein a fresh feedstream comprising isobutylene and methanol is fed through said first reaction zone at an LHSV of 2 to 5, yielding an isobutylene conversion of at least 90 weight percent and at least as great as it would be under the same inlet temperature, adiabatic and fresh feed LHSV conditions without effluent recycling and effluent is recycled from and through said first reaction zone along with said fresh feedstream, the recycle ratio of effluent from said first reaction zone being not greater than 14, and unrecycled effluent from said first reaction zone is passed through said second reaction zone with an inlet temperature in the range of 38 to 43°C and at a LHSV in the range of 3 to 7, yielding a conversion of at least 50 weight percent of the remaining isobutylene characterized in that the molar ratio of methanol to isobutylene in the combined feeds entering said first reaction zone is in the range of 0.99:1 to 1.02:1 and the inlet temperature of the feeds is in the range of 49 to 60°C.

2. A process according to claim 1 characterized in that the LHSV of effluent passed through said second reaction zone is such that the hottest spot in the catalyst bed is no greater than 6°C above the inlet temperature of said effluent into said second reaction zone.

3. A process according to claim 1 or 2 characterized in that said fresh feed stream contains no more than 25 weight percent isobutylene on a methanol free basis.

4. A process according to claim 3 characterized in that said fresh feed stream contains from 10 to 20 weight percent isobutylene on a methanol free basis.

5. A process according to claim 4 characterized in that said fresh feedstream contains isobutylene in the range of 15 to 20 weight percent on a methanol free basis.

6. A process according to any of the preceding claims characterized in that said fresh feedstream comprises the fraction of the hydrocarbons from a stream or catalytic cracking process that boils in the range of $C_4$ hydrocarbons.

7. A process according to any of the preceding claims characterized in that the recycle ratio of effluent from said first reaction zone is from 2 to 6.

8. A process according to claim 7 characterized in that the recycle ratio of effluent from said first reaction zone is no greater than 5.

9. A process according to any of the preceding claims characterized in that the LHSV of the fresh feedstream through said first reaction zone is in the range of 3 to 5.

10. A process according to any of the preceding claims characterized in that the LHSV of the fresh feedstream is 5, the recycle ratio is in the range of 1.5 to 3, and the inlet temperature to said first reaction zone is 54°C.

11. process according to any of the preceding claims characterized in that the effluent from said second reaction zone is subjected to distillation to obtain an MTBE product substantially free of methanol.

**Patentansprüche**

1. Verfahren zur Herstellung von Methyltertiätbutylether durch Umsetzen von Isobutylen und Methanol, wobei die Umsetzung in zwei Stufen in einer ersten und einer zweiten Reaktionszone durchgeführt wird, von denen jede ein fixiertes Bett von saurem Ionenaustauscherharz aufweist, und wobei die Umsetzung unter im wesentlichen adiabatischen Bedingungen und bei einem zur Aufrechterhaltung der Reaktionspartner in flüssiger Phase ausreichenden Druck gehalten wird, wobei ein frischer, Isobutylen und Methanol enthaltenden Beschickungsstrom mit einem Raumdurchsatz (Flüssigkeit pro Stunde) von 2 bis 5 durch die erste Reaktionszone geschickt wird und dabei eine Isobutylenumwandlung ergibt, die mindestens 90 Gewichtsprozent beträgt und und mindestens so groß ist, wie sie unter gleicen adiabatischen Bedingungen und gleichen Bedingungen von Einlaßtemperatur und Raumdurchsatz der frischen Beschickung ohne Ablaufrückführung wäre, und wobei der Ablauf aus und durch die erste Reaktionszone zusammen mit dem frischen Beschickungsstrom mit einem Rückführungsverhältnis von Ablauf aus der ersten Reaktionszone von nicht größer als 14 rückgeführt wird, und wobei nicht rückgeführter Ablauf aus der ersten Reaktionszone durch die zweite Reaktionszone mit einer Einlaßtemperatur im Bereich von 38 bis 43°C und mit einerm Raumdurchsatz im Bereich von 3 bis 7 geleitet wird und dabei eine Umwandlung von zumindest 50 Gewichtsprozent der verbleibenden Isobutylens liefert, dadurch gekennzeichnet, daß das Molverhältnis von Methanol zu Izobutylen in den vereinigten Beschickungen beim Eintritt in die erste Reaktionszone im Bereich von 0.99:1 bis 1,02:1 liegt, und daß die Einlaßtemperatur der Beschickungen im Bereich von 49 bis 60°C liegt.

2. Verfahren nach Anspruch 1, dadurch gekennzeichnet, daß man den Raumdurchsatz des durch die zweite Reaktionszone geleiteten Ablaufes so wählt, daß der heißeste Punkt in dem Katalysatorbett nicht mehr als 6°C über der Einlaßtemperatur des Ablaufes in die zweite Reaktionszone liegt.

3. Verfahren nach Anspruch 1 oder 2, dadurch gekennzeichnet, daß der frische Beschickungsstrom nicht mehr als 25 Gewichtsprozent Isobutylen auf methanolfreier Basis enthält.

4. Verfahren nach Anspruch 3, dadurch gekennzeichnet, daß der frische Beschickungsstrom von 10 bis 20 Gewichtsprozent Isobutylen auf metanolfreier Basis enthält.

5. Verfahren nach Anspruch 4, dadurch gekennzeichnet, daß der frische Beschickungsstrom Isobutylen im Bereich von 15 bis 20 Gewichtsprozent auf methanolfreier Basis enthält.

6. Verfahren nach einem der vorhergehenden Ansprüche, dadurch gekennzeichnet, daß der frische Beschickungsstrom die Fraktion der Kohlenwasserstoffe aus einem Strom oder einem katalytischen Crackprozess enthält, die im Bereich der $C_4$—Kohlenwasserstoffe siedet.

7. Verfahren nach einem der vorhergehenden Ansprüche, dadurch gekennzeichnet, daß das Rückführungsverhältnis des Ablaufs aus der ersten Reaktionszone von 2 bis 6 beträgt.

8. Verfahren nach Anspruch 7, dadurch gekennzeichnet, daß das Rückführungsverhältnis des Ablaufs aus der ersten Reaktionszone nicht größer als 5 ist.

9. Verfahren nach einem der vorhergehenden Ansprüche, dadurch gekennzeichnet, daß der Raumdurchsatz des frischen Beschickungsstromes durch die erste Reaktionszone im Bereich von 3 bis 5 liegt.

10. Verfahren nach einem der vorhergehenden Ansprüche, dadurch gekennzeichnet, daß der Raumdurchsatz des frischen Beschickungsstromes 5 ist, das Rückführungsverhältnis im Bereich von 1,5 bis 3 liegt, und daß die Einlaßtemperatur zu ersten Reaktionszone 54°C beträgt.

11. Verfahren nach einem der vorhergehenden Ansprüche, dadurch gekennzeichnet, daß man den Ablauf aus der zweiten Reaktionszone zur Gewinnung eines im wesentlichen methanolfreien MTBE-Produktes der Destilation unterwirft.

## Revendications

1. Procédé de préparation de l'éther méthyl tertbutylique en faisant réagir de l'isobutylène et du méthanol dans lequel cette réaction est effectué en deux stades dans une première et une seconde zones réactionnelles comprenant chacune un lit fixe de résine échangeuse d'ions acide et maintenues dans des conditions pratiquement adiabatiques et sous une pression suffisante pour maintenir les réactifs en phase liquide, dans lequel un courant d'alimentation frais comprenant de l'isobutylène et du méthanol est envoyé à travers cette premièr zone réactionnelle à une vitesse spatiale liquide horaire de 2 à 5, en donnant un taux de conversion de l'isobutylène d'au moins 90% en poids et au moins aussi élevé qu'il serait à la même température d'entrée, dans les mêmes conditions adiabatiques et de vitesse spatiale liquide horaire de l'alimentation fraîche sans recyclage de l'effluent et l'effluent est recyclé à partir de et à travers cette première zone réactionnelle en même temps que ce courant d'alimentation frais, le rapport de recyclage de l'effluent de cette première zone réactionnelle n'étant pas supérieur à 14, et de l'effluent non recyclé provenant de cette première zone réactionnelle est envoyé à travers cette seconde zone réactionnelle avec une température d'entrée dans l'intervalle de 38 à 43°C et à une vitesse spatiale liquide horaire dans l'intervalle de 3 à 7, donnant un taux de conversion d'au moins 50% en poids de l'isobutylène restant, caractérisé en ce que le rapport molaire du méthanol à l'isobutylène dans les alimentations combinées pénétrant dans cette première zone réactionnelle est dans l'intervalle de 0,99:1 à 1,02:1, et en ce que la température d'entrée des alimentations est dans l'intervalle de 49 à 60°C.

2. Procédé selon la revendication 1, caractérisé en ce que la vitesse spatiale liquide horaire de l'effluent envoye à travers cette seconde zone réactionnelle est telle que le point le plus chaud du lit de catalyseur ne soit pas supérieur de plus de 6°C à la température d'entrée de cet effluent dans cette seconde zone réactionnelle.

3. Procédé selon la revendication 1 ou 2, caractérisé en ce que ce courant d'alimentation frais ne contient pas plus de 25% en poids d'isobutylène sur une base exempte de méthanol.

4. Procédé selon la revendication 3, caractérisé en ce que ce courant d'alimentation fraise contient de 10 à 20% en poids d'isobutylène sur une base exempte de méthanol.

5. Procédé selon la revendication 4, caractérisé en ce que ce courant d'alimentation frais contient de l'isobutylène dans l'intervalle de 15 à 20% en poids sur une base exempte de méthanol.

6. Procédé selon l'une quelconque des revendications précédentes, caractérisé en ce que ce courant d'alimentation frais comprend la fraction des hydrocarbures d'un courant ou d'un procédé de craquage catalytique qui bout dans l'intervalle des hydrocarbures en $C_4$.

7. Procédé selon l'une quelconque des revendications précédentes, caractérisé en ce que le rapport de recyclage de l'effluent provenant de cette première zone réactionnelle est de 2 à 6.

8. Procédé selon la revendication 7, caractérisé en ce que le rapport de recyclage de l'effluent provenant de cette première zone réactionnelle n'est pas supérieur à 5.

9. Procédé selon l'une quelconque des revendications précédentes, caractérisé en ce que la vitesse spatiale liquide horaire du courant d'alimentation frais à travers cette première zone réactionnelle est dans l'intervalle de 3 à 5.

10. Procédé selon l'une quelconque des revendications précédentes, caractérisé en ce que la vitesse spatiale liquide horaire du courant d'alimentation frais est de 5, le rapport de recyclage est dans l'intervalle de 1,5 à 3, et la température d'entrée dans cette première zone réactionnelle est de 54°C.

11. Procédé selon l'une quelconque des revendications précédentes, caractérisé en ce que l'effluent sortant de cette seconde zone réactionnelle est soumis à une distillation pour obtenir un MTBE pratiquement exempt de méthanol.

FIG. 1

FIG. 2

2

FIG. 3

2ND STAGE REACTOR SIMULATION

FIG. 4

FIG. 5

FIG. 6

6

FIG. 7

FIG. 8

8

# 0 071 238

CATALYST: AMBERLYST 15
FEED LHSV, HR$^{-1}$=3.0
INLET TEMP., °F=130
REACTOR PRESSURE ≈ 160 PSIG

MeOH/iC$_4$=
MOLAR RATIO

FIG. 9

9

FIG. 10